Europäisches Patentamt

(19) European Patent Office

Office européen des brevets

(11) Veröffentlichungsnummer: **0 110 475**
**B1**

(12) **EUROPÄISCHE PATENTSCHRIFT**

(45) Veröffentlichungstag der Patentschrift:
18.02.87

(51) Int. Cl.⁴: **A 61 B 6/04,** A 61 G 13/00

(21) Anmeldenummer: 83201675.2

(22) Anmeldetag: 29.11.83

(54) Röntgengerät mit einer in ihrer Längsrichtung verschiebbaren Tischplatte.

(30) Priorität: 02.12.82 DE 3244579

(43) Veröffentlichungstag der Anmeldung:
13.06.84 Patentblatt 84/24

(45) Bekanntmachung des Hinweises auf die Patenterteilung:
18.02.87 Patentblatt 87/8

(84) Benannte Vertragsstaaten:
BE DE FR GB IT

(56) Entgegenhaltungen:
CH - A - 319 407
FR - A - 1 400 490
US - A - 3 472 085
US - A - 3 473 024
US - A - 3 588 500
US - A - 3 944 204
US - A - 4 105 923
US - A - 4 164 656

ANTRIEBSTECHNIK, Band 5, Nr. 1, Januar 1966, Seiten 14-17, Krausskopf-VLG, Mainz, DE,
"Präzisions-Rollenketten und ihre vielseitige Verwendung"

(73) Patentinhaber: Philips Patentverwaltung GmbH,
Billstrasse 80, D-2000 Hamburg 28 (DE)

(84) Benannte Vertragsstaaten: DE

(73) Patentinhaber: N.V. Philips' Gloeilampenfabrieken,
Groenewoudseweg 1, NL-5621 BA Eindhoven (NL)

(84) Benannte Vertragsstaaten: BE FR GB IT

(72) Erfinder: Wallis, Manfred, Beim Schlump 30,
D-2000 Hamburg 13 (DE)

(74) Vertreter: Hartmann, Heinrich, Dipl.-Ing. et al, Philips
Patentverwaltung GmbH
Billstrasse 80 Postfach 10 51 49,
D-2000 Hamburg 28 (DE)

## Beschreibung

Die Erfindung betrifft ein Röntgengerät nach dem Oberbegriff eines der Patentansprüche 1 oder 2.

Aus der US-PS 3 473 024 ist ein Röntgengerät bekannt, bei dem die Kopplung zwischen der Kette und der Tischplatte über ein Koppelstück erfolgt, das in die Kette eingesetzt und fest mit der Tischplatte verbunden ist. Ein Nachteil des bekannten Röntgengerätes besteht darin, dass der Hub der Tischplatte (das ist die Strecke, um die die Tischplatte in Längsrichtung verschiebbar ist) kleiner ist als der Abstand der Kettenräder. Der Hub war daher in der Praxis bisher auf ca. 1,50 m begrenzt.

Dieser Nachteil gilt in gleicher Weise für das aus der US-PS 3 588 500 bekannte Röntgengerät mit einer in ihrer Längsrichtung verschiebbaren Tischplatte, die über in Tischlängsrichtung gegeneinander versetzt angeordnete Koppelelemente kammartig in eine endlose Kette eingreifen, die über in Längsrichtung versetzte Kettenräder geführt ist, von denen eins motorisch antreibbar ist. Die Koppelelemente sind dabei Teil eines Rastmechanismus, der bei horizontaler Stellung des Röntgenuntersuchungsgerätes in die Kette ein- bzw. ausgerastet werden kann, so dass die Tischplatte wahlweise von Hand oder motorisch verschoben werden kann. Auch für dieses bekannte Gerät gilt aber die Beschränkung, dass der Hub der Tischplatte kleiner ist als der Abstand der Kettenräder.

Aufgabe der vorliegenden Erfindung ist es, ein Röntgengerät eingangs genannter Art so auszugestalten, dass bei gleichem Abstand der Kettenräder ein grösserer Hub erzielbar ist.

Eine erste Lösung dieser Aufgabe ist in Anspruch 1 angegeben und eine zweite Lösung in Anspruch 2. Dabei kann theoretisch die Tischplatte so weit über das Kopf- bzw. das Fussende hinaus verschoben werden, bis nur noch wenigstens ein Koppelelement in die Kette eingreift.

Die Erfindung wird nachstehend anhand der Zeichnung näher erläutert. Es zeigen:

Fig. 1 ein Röntgengerät, bei dem die Erfindung anwendbar ist,

Fig. 2 eine Einzelheit dieses Gerätes in perspektivischer Darstellung, und

Fig. 3 eine bevorzugte Ausführungsform der Koppelelemente.

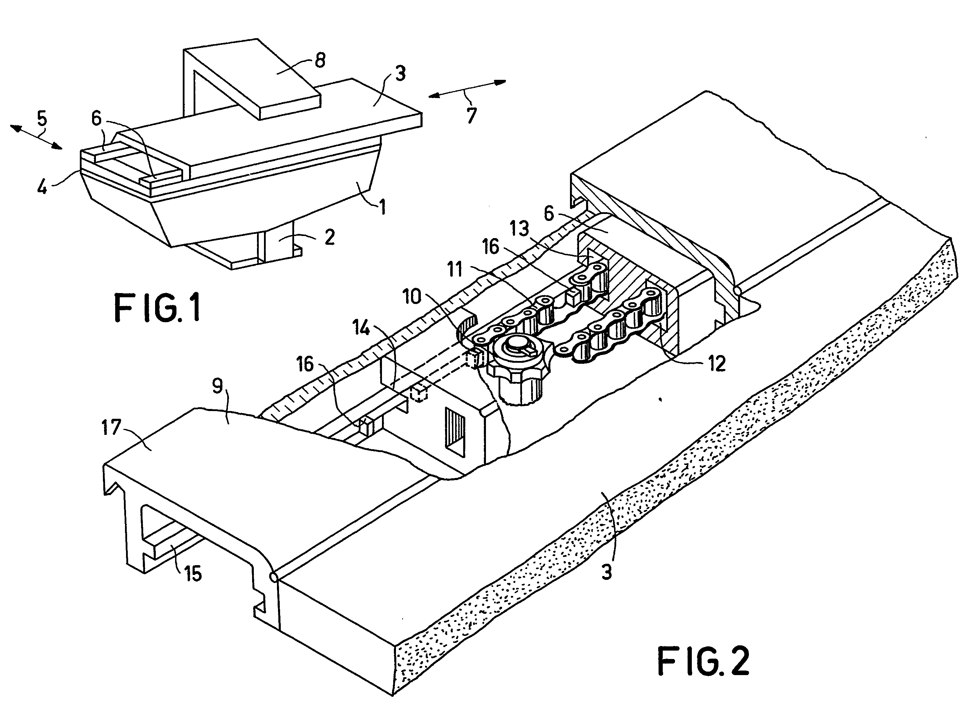

In Fig. 1 ist ein Röntgenuntersuchungsgerät dargestellt, dessen Rahmen 1 um eine horizontale Achse schwenkbar in einer Fusskonstruktion 2 gelagert ist. Der Rahmen trägt einen parallel zur Schwenkachse, d.h. in Richtung des Doppelpfeils 5, verschiebbaren Querwagen 4, der mit zwei parallelen Führungsschienen 6 versehen ist, die senkrecht zur Schwenkachse verlaufen und zur Führung einer Tischplatte 3 in deren Längsrichtung, d.h. in Richtung des Doppelpfeiles 7, dienen. Ausserdem umfasst das Röntgenuntersuchungsgerät noch ein Röntgenzielgerät 8. Die Verschiebung der Tischplatte 3 erfolgt mit Hilfe einer in Fig. 1 nicht näher dargestellten Kette, auf die über ein Kettenrad ein Motor – beides in Fig. 1 ebenfalls nicht dargestellt – einwirkt.

Wie in Fig. 2 angedeutet, ist in jeder der Führungsschienen 6 jeweils in der Nähe ihrer beiden Enden je ein Kettenrad 10 angeordnet, dessen Drehachse senkrecht zur Ebene der Tischplatte verläuft. Um die beiden Kettenräder 10 in einer Führungsschiene 6 ist eine endlose Kette gespannt, die in Kanälen 12 und 13 geführt ist, die in der Führungsschiene in Längsrichtung verlaufen. Der Kanal 13, d.h. der äussere der beiden Kanäle, ist auf seiner Aussenseite durch einen Schlitz 14 geöffnet, der sich in Längsrichtung über die gesamte Länge der Führungsschiene 6 erstreckt. In diesen Schlitz ragt eine in seiner Längsrichtung verlaufende Zahnstange 15 hinein, von deren Zähnen 16 zumindest einige in die Kette 11 eingreifen, mit dieser jedoch nicht fest verbunden sind. Die Zahnstange 15 ist Teil einer Profilschiene 9, mit der die Tischplatte 3 an ihrer Längskante versehen ist. Die Verschiebung der Tischplatte in Längsrichtung erfolgt dadurch, dass eines der erwähnten Kettenräder, z.B. das Kettenrad 10, durch den nicht näher dargestellten Motor angetrieben wird. Die von dem Kettenrad auf die Kette 11 ausgeübte Kraft wird auf die Zähne 16 über die Tischplatte 3 übertragen, die infolgedessen in Längsrichtung verschoben wird.

Wenn die Zahnstange relativ lang ist oder die Tischplatte weit in Kopf- oder in Fussrichtung verschoben wird, besteht die Gefahr, dass die Zähne der Zahnstange mit den Zähnen des Kettenrades kollidieren. Dieser Gefahr könnte dadurch begegnet werden, dass die Kettenräder einerseits und die Zahnstange andererseits in Richtung senkrecht zur Tischplatte versetzt werden, so dass ihre Zähne in verschiedenen zur Ebene der Tischplatte 3 parallelen Ebenen verlaufen. Eine bevorzugte Lösung dieses Problems besteht jedoch darin, dass der Abstand zweier benachbarter Zähne auf der Zahnstange das Doppelte der Kettenteilung (das ist die Länge der Kette dividiert durch die Zahl der Kettenglieder) beträgt und dass die Zähne der Kettenräder so auf deren Umfang versetzt sind, dass sie nur in jedes zweite Kettenglied eingreifen. Wird die Zahnstange nun so angebracht, dass sie nur in die Kettenglieder eingreifen, in die die Zähne der Kettenräder nicht eingreifen können, ist die Gefahr einer Kollision zwischen den Zähnen vermieden. Grundsätzlich könnte der Abstand der Zähne auf der Zahnstange auch das Vierfache einer Kettenteilung betragen. Ebenso ist es möglich, nur soviel Zähne auf den Kettenrädern vorzusehen, dass nur in jedes dritte Kettenglied ein Zahn eingreifen kann. In diesem Fall dürften die Zähne der Zahnstange ebenfalls nur in jedes dritte (oder sechste oder neunte) Kettenglied eingreifen.

An sich entstehen durch die beschriebene Art der Kopplung zwischen Tischplatte und Kette Kräfte, die die Kette senkrecht zu ihrer Bewegungsrichtung auslenken. Die Führung der Kette 11 in den Kanälen 12 und 13 verhindert jedoch, dass diese so weit ausgelenkt werden kann, dass die Zahnstange nicht mehr mit ihr in Eingriff steht.

Auch die andere Führungsschiene ist genauso ausgebildet wie die in Fig. 2 dargestellte, jedoch spiegelbildlich zu dieser angeordnet und wird von einer weiteren, ebenfalls identisch aufgebauten und spiegelbildlich angeordneten Profilschiene an der anderen Längskante der Tischplatte umschlossen. Die Kette in dieser anderen Führungsschiene wird mit der gleichen Geschwindigkeit angetrieben wie die in Fig. 2 dargestellte Kette, jedoch mit entgegengesetztem Umlaufsinn. Die Führungsschienen erfüllen dabei drei Funktionen: Sie führen die Ketten, dienen ihrerseits als Gleitführung für die Profilschienen und damit für die Tischplatte, und verhindern das Abheben der Tischplatte durch das Eingreifen der Stege 15 in die Schlitze 14.

Durch die Erfindung ist der Hub der Tischplatte um fast das Doppelte des Abstandes der beiden äussersten Koppelelemente vergrösserbar, was in der Praxis jedoch nicht voll ausgenutzt wird.

**Patentansprüche**

1. Röntgengerät mit einer in ihrer Längsrichtung verschiebbaren Tischplatte (3), die über in Tischlängsrichtung gegeneinander versetzt angeordnete Koppelelemente (16) kammartig in eine endlose Kette (11) eingreift, die über in Tischlängsrichtung gegeneinander versetzte Kettenräder geführt ist, von denen eines motorisch antreibbar ist, dadurch gekennzeichnet, dass der Abstand zweier benachbarter Koppelelemente (16) n mal so gross ist wie die Kettenteilung, wobei n eine natürliche Zahl grösser als oder gleich 2 ist, dass die Zähne der Kettenräder (10) so auf deren Umfang versetzt sind, dass sie nur in jedes n-te Kettenglied eingreifen und dass die Koppelelemente und die Zähne der Kettenräder so angeordnet sind, dass sie gegeneinander versetzt in die Kette eingreifen.

2. Röntgengerät mit einer in ihrer Längsrichtung verschiebbaren Tischplatte (3), die über in Tischlängsrichtung gegeneinander versetzt angeordnete Koppelelemente (16) kammartig in eine endlose Kette (11) eingreift, die über in Tischlängsrichtung gegeneinander versetzte Kettenräder geführt ist, von denen eines motorisch antreibbar ist, dadurch gekennzeichnet, dass die Zähne der Kettenräder (10) einerseits und die Koppelelemente (16) andererseits in verschiedenen zueinander parallelen Ebenen in die Kette (11) eingreifen.

3. Röntgengerät nach einem der Ansprüche 1 oder 2, dadurch gekennzeichnet, dass die Koppelelemente (16) Teil einer in Längsrichtung verlaufenden, mit der Tischplatte (3) fest verbundenen Zahnstange (15) sind.

4. Röntgengerät nach einem der vorhergehenden Ansprüche, dadurch gekennzeichnet, dass Führungsmittel (6, 12, 13) vorgesehen sind, die die Auslenkung zumindest des mit den Koppelelementen (16) zusammenwirkenden Teils der Kette (11) senkrecht zur Längsrichtung begrenzen.

5. Röntgengerät nach Anspruch 4, dadurch gekennzeichnet, dass die Tischplatte durch zwei synchron laufende Ketten (11) angetrieben wird, die mit je einem Satz von seitlich an der Tischplatte (3) angebrachten Koppelelementen zusammenwirken, dass jede Kette in den Kanälen (12, 13) einer Führungsschiene (6) geführt ist, dass jede Führungsschiene mit einem seitlich und in Längsrichtung verlaufenden Schlitz (14) versehen ist, durch den hindurch die Koppelelemente (16) mit der Tischplatte (3) in Verbindung stehen.

6. Röntgengerät nach Anspruch 5, dadurch gekennzeichnet, dass die Tischplatte an ihren beiden Längskanten Profilschienen (15) aufweist, die je eine Führungsschiene (6) umschliessen und auf der zugehörigen Führungsschiene gleitend geführt sind.

**Claims**

1. An X-ray apparatus comprising a table-top (3) which is displaceable in the longitudinal direction and which engages an endless chain (11) like a comb via coupling elements (16) which are arranged so as to be offset with respect to one another in the longitudinal direction of the table-top, which chain is guided by sprockets which are arranged so as to be offset with respect to one another in the longitudinal direction of the table-top and one of which can be driven by a motor, characterized in that the distance between two adjacent coupling elements (16) amounts to n times the pitch of the chain, n being a natural number larger than or equal to 2, the teeth of the sprockets (10) being arranged so as to be offset on the circumference thereof so that they engage only every $n^{th}$ chain link, the coupling elements and the teeth of the sprockets being arranged so that they engage the chain in an offset manner with respect to one another.

2. An X-ray apparatus comprising a table-top (3) which is displaceable in the longitudinal direction and which engages an endless chain (11) like a comb via coupling elements (16) which are arranged so as to be offset with respect to one another in the longitudinal direction of the table-top, which chain is guided by sprockets which are arranged so as to be offset with respect to one another in the longitudinal direction of the table-top and one of which can be driven by a motor, characterized in that the teeth of the sprockets (10) engage the chain (11) on the one side and the coupling elements (16) engage the chain on the other side in different mutually parallel planes.

3. An X-ray apparatus as claimed in Claim 1 or 2, characterized in that the coupling elements (16) form part of a toothed rack (15) which is arranged in the longitudinal direction and which is rigidly connected to the table-top (3).

4. An X-ray apparatus as claimed in any one of the preceding Claims, characterized in that there are provided guide means (6, 12, 13) which limit the deflection of at least the part of the chain (11) which cooperates with the coupling elements (16) in the direction perpendicular to the longitudinal direction.

5. An X-ray apparatus as claimed in Claim 4, characterized in that the table-top is driven by two synchronized chains (11), each of which cooperates with a set of coupling elements which are laterally provided on the table-top (3), each chain being guided in the ducts (12, 13) of a guide rail (6), each guide rail being provided with a slit (14) which extends laterally and in the longitudinal direction and wherethrough the coupling elements (16) engage the table-top (3).

6. An X-ray apparatus as claimed in Claim 5, characterized in that at both its long sides the table-top is provided with profiled rails (15), each of which encloses a guide rail (6) and is guided so as to be slidable on the associated guide rail.

**Revendications**

1. Appareil à rayons X comportant une table (3) déplaçable dans le sens de sa longueur, qui vient en prise par l'intermédiaire d'éléments de couplage (16) décalés les uns des autres dans le sens longitudinal de la table, à la façon d'un peigne, avec une chaîne sans fin (11) qui est guidée par l'intermédiaire de roues à chaîne décalées l'une de l'autre dans le sens longitudinal de la table, une de ces roues pouvant être entraînée par un moteur, caractérisé en ce que la distance entre deux éléments de couplage (16) voisins vaut n fois le pas de la chaîne, n étant un nombre naturel qui est supérieur ou égal à 2, les dents des roues à chaîne (10) sont décalées sur le pourtour des roues d'une manière telle qu'elles ne s'engagent que dans chaque $n^{\text{ième}}$ maillon de la chaîne et les éléments de couplage ainsi que les dents des roues à chaîne sont disposés d'une manière telle qu'ils viennent en prise avec la chaîne à des endroits décalés les uns des autres.

2. Appareil à rayons X comportant une table (3) déplaçable dans le sens de sa longueur qui vient en prise par l'intermédiaire d'éléments de couplage (16) décalés l'un de l'autre dans le sens longitudinal de la table, à la manière d'un peigne, avec une chaîne sans fin (11) qui est guidée par l'intermédiaire de roues à chaîne décalées l'une de l'autre dans le sens longitudinal de la table, une de ces roues à chaîne pouvant être entraînée par un moteur, caractérisé en ce que les dents des roues à chaîne (10) d'une part et les éléments de couplage (16) d'autre part viennent en prise avec la chaîne (11) dans des plans différents parallèles l'un à l'autre.

3. Appareil à rayons X suivant la revendication 1 ou 2, caractérisé en ce que les éléments de couplage (16) font partie d'une crémaillère (15) s'étendant dans le sens longitudinal et reliée rigidement à la table (3).

4. Appareil à rayons X suivant l'une quelconque des revendications précédentes, caractérisé en ce que des moyens de guidage (6, 12, 13) sont prévus qui limitent la déviation, dans un sens perpendiculaire à la direction longitudinale de la chaîne, d'au moins la partie de la chaîne (11) coopérant avec les éléments de couplage (16).

5. Appareil à rayons X suivant la revendication 4, caractérisé en ce que la table est entraînée par deux chaînes (11) qui se déplacent de manière synchrone qui coopèrent chacune avec un jeu d'éléments de couplage disposés latéralement sur la table (3), chaque chaîne est guidée dans des canaux (12, 13) d'un rail de guidage (6), chaque rail de guidage est pourvu d'une fente (14) qui s'étend latéralement et dans le sens longitudinal, à travers laquelle les éléments de couplage (16) sont en liaison avec la table (3).

6. Appareil à rayons X suivant la revendication 5, caractérisé en ce que la table présente sur ses deux rives longitudinales (15) des glissières profilées qui enserrent chacune un rail de guidage (6) et sont guidées à glissement sur leur rail de guidage associé.

FIG.1

FIG.2

0 110 475